# NEUE EUROPÄISCHE PATENTSCHRIFT

(11) **EP 1 089 748 B2**
(45) Veröffentlichungstag und Bekanntmachung des Hinweises auf die Entscheidung über den Einspruch: **10.11.2010**
(45) Hinweis auf die Patenterteilung: 04.06.2003
(21) Anmeldenummer: 99939923.1
(22) Anmeldetag: 19.06.1999
(51) Int. Cl.: A61K 36/16, A61Q 19/00, A23L 1/00

(54) **WASSERLÖSLICHER NATIVER TROCKENEXTRAKT AUS GINKGO BILOBA MIT HOHEM GEHALT AN TERPENOIDEN UND FLAVONGLYKOSIDEN**
WATER-SOLUBLE NATIVE DRY GINKGO BILOBA EXTRACT WITH A HIGH TERPENOID AND FLAVONE GLYCOSIDE CONTENT
EXTRAIT SEC HYDROSOLUBLE DE GINKGO BILOBA PRESENTANT UNE TENEUR ELEVEE EN TERPENOIDES ET FLAVONGLYCOSIDES

(30) Priorität: 02.07.1998 DE 19829516
(43) Veröffentlichungstag der Anmeldung: 11.04.2001
(73) Patentinhaber: Dr. Willmar Schwabe GmbH & Co., D-76209 Karlsruhe (DE)
(72) Erfinder: OSCHMANN, Rainer, D-76829 Landau (DE); GRETHLEIN, Eckhardt, D-76327 Pfinztal (DE); Stumpf, Karl-Heinz Dr., 76228 Karlsruhe (DE); Erdelmeier, Clemens Dr., 76139 Karlsruhe (DE); Sudeck, Armin, 76646 Bruchsal (DE)
(74) Vertreter: Rudolph, Ulrike
(86) Internationale Anmeldenummer: PCT/DE1999/001812
(87) Internationale Veröffentlichungsnummer: WO 2000/001397

(56) Entgegenhaltungen:
- EP-A- 0 324 197
- EP-A- 0 330 567
- EP-A- 0 360 556
- DE-A- 3 940 092
- DE-B2- 2 117 429
- DE-C2- 3 940 094
- BIOLOGICAL ABSTRACTS, vol. 105, Philadelphia, PA, US; abstract no. 199800309451, LI XIN-GANG ET AL.: "PREPARATION OF DRY EXTRACT RICH IN GINKGOLIDES FROM GINKGO BILOBA L. LEAVES." XP002122095 & ZHONGGUO YIYAO GONGYE ZAZHI, Bd. 29, Nr. 1, Januar 1998 (1998-01), Seiten 8-9,
- DATABASE WPI Section Ch, Week 199751 Derwent Publications Ltd., London, GB; Class E13, AN 1997-550558 XP002122096 & CN 1 129 218 A (GENG P), 21. August 1996 (1996-08-21)
- PATENT ABSTRACTS OF JAPAN vol. 1995, no. 01, 28. Februar 1995 (1995-02-28) & JP 06 279300 A (NIPPON GREEN UEEBU KK;OTHERS: 01), 4. Oktober 1994 (1994-10-04) in der Anmeldung erwähnt
- Jap. Patent Appl. No. Hei 4 - 321616

## Beschreibung

Die Erfindung betrifft ein Verfahren zur Herstellung eines wasserlöslichen, nativen Trockenextrakts aus Blättern von *Ginkgo biloba* sowie den damit hergestellten Trockenextrakt.

"Wasserlöslich" heißt im folgenden: in purem Wasser löslich und "leicht löslich" gemäß der Definition im "Europäischen Arzneibuch" 1997, 3. Ausgabe (amtliche deutsche Ausgabe, Deutscher Apotheker Verlag Stuttgart, Govi-Verlag-Pharmazeutischer Verlag GmbH Eschborn)

Zubereitungen auf der Basis von Extrakten aus *Ginkgo biloba* Blättern finden eine vielfältige Anwendung in medizinischen und kosmetischen Bereichen. Die pharmazeutische Wirkung der *Ginkgo biloba* Extrakte ist vor allem auf die Inhaltsstoffe Ginkgoflavonglykoside und Terpenoide (wie Ginkgolide, Bilobalide) zurückzuführen.

Die Herstellung von *Ginkgo biloba* Extrakten kann auf vielfältige Weise erfolgen. Nach einem gängigen Grundverfahrensprinzip werden zunächst *Ginkgo biloba* Blätter mit einem Extraktionsmittel, bestehend aus einer wässrigen Lösung eines niederen aliphatischen Ketons oder eines Alkohols, extrahiert. Der hierbei erhaltene Rohextrakt wird zwecks Aufreinigung einer Fällungsreaktion mit Wasser in der Kälte unterworfen, und die ausgefallenen Abfallprodukte, insbesondere lipophile Bestandteile, werden entfernt.
Zur weitergehenden Aufreinigung dieses Rohextrakts mit dem Ziel, gewünschte Inhaltsstoffgruppen anzureichern, sind im Stand der Technik verschiedene Verfahren bekannt. So wird z.B. gemäß der DE 39 40 091 C2 eine Bleifallung durchgeführt, die zwar zur Entfernung vieler unerwünschter Komponenten führt, die aber die mit dem Einsatz von Blei verbundenen Nachteile, insbesondere das Gesundheitsrisiko für die damit arbeitenden Personen und relativ hohe Kosten, mit sich bringt
Die DE 39 40 092 C2 schlägt anstelle der Bleifällung eine Extraktion des Primarextrakts mit n-Butanol in Wasser vor, und die US 5 637 302 eine Extraktion mit n-Butanol und Toluol. Mit diesen Maßnahmen geht der Nachteil der Verwendung organischer Lösungsmittel mit gesundheitlichem Gefährdungspotential einher.
In der J 27 93 00/1994 wird der Rohextrakt zur Anreicherung der gewünschten Wertstoffe an polare Adsorberharze adsorbiert
Bei allen diesen beschriebenen Extrakten sind die hinsichtlich der pharmakologischen Wirksamkeit relevanten Inhaltsstoffe angereichert, und zwar üblicherweise derart, daß das Verhaltnis von Droge zu Extrakt 30 bis 70 zu 1 beträgt. Die aus diesen Herstellungsverfahren resultierenden Ginkgo biloba Trockenextrakte weisen alle eine schlechte Wasserloslichkeit auf Diese Extrakte werden deshalb häufig noch einer weiteren Behandlung unterworfen, um ihre bekanntermaßen schlechte Wasserloslichkeit zu verbessern Hierfür sind im Stand der Technik ebenfalls verschiedene Verfahren bekannt, die aber allesamt nur Kompromißlösungen darstellen
Die EP 0 764 659 A1 beschreibt ein Verfahren zur Verbesserung der extrem geringen Wasserlöslichkeit der therapeutisch besonders wichtigen Ginkoliden (Löslichkeit unter 0,02%), das durch die Durchführung einer Komplexbildungsreaktion zwischen den Ginkgoliden und Cyclodextrinen charakterisiert ist und zu Ginkgolid-Cyclodextrin-Komplexen führt, die gut mit Wasser in Losung gehen. Dieses Komplexbildungsverfahren ist jedoch technisch sehr aufwendig
Aus der DE 43 34 600 C2 ist die Verwendung von Dimethylisosorbid und Polyalkohol als Solubilisierungshilfsmittel für *Ginkgo biloba* Extrakte in wässriger Lösung bzw in einer Wasser-Öl-Emulsion bekannt.
In der EP 0 577 143 A2 wird allgemein zur Verbesserung der Wasserlöslichkeit von schwer wasserlöslichen Flavonoiden vorgeschlagen, die Flavonoide molekulardispers in einer Basissubstanz aus hydrophilem Peptid mit einem Molekulargewicht größer 100 Dalton, insbesondere Gelatine, zu verteilen, und sie auf diese Weise in einer stabilen festen oder flüssigen Losung zu halten

Ein alternatives Verfahren ist in der EP 0 275 005 beschrieben, nämlich die Umsetzung von Flavonoiden mit Phospholipiden als Solubilisierungshilfsmittel.
Und aus der WO 96/29085 ist schließlich eine *Ginkgo biloba* Trockenextrakt-Zubereitung bekannt, die als Solubilisierungshilfsmittel ein Brausegemisch aus einer physiologisch verträglichen Säure bzw. deren Natriumsalz und einem physiologisch verträglichen Carbonat bzw. Hydrogencarbonat enthält.

Die genannten Verfahren aus dem Stand der Technik haben alle insbesondere den Nachteil, daß spezielle Solubilisierungshilfsmittel oder andere galenische Hilfsstoffe während oder nach der Herstellung des Extrakts eingesetzt werden. Diese Hilfsstoffe sind letztendlich auch in der endgültigen Wirkstoffzubereitung enthalten und dort nicht immer erwünscht oder sogar z.T. erheblich störend (z.B. weil sie die Ginkgolide in Komplexen binden und damit deren Freisetzung behindern).

Aufgabe der vorliegenden Erfindung ist die Bereitstellung eines Verfahrens zur Herstellung eines nativen Trockenextrakts aus Blättern von *Ginkgo biloba,* der vollständig wasserlöslich ist und einen hohen Gehalt an Terpenoiden und Flavonglykosiden aufweist, sowie die Bereitstellung eines solchen Trockenextrakts.

Diese Aufgabe wird mit der Bereitstellung eines Verfahrens der eingangs genannten Art gelöst, welches dadurch gekennzeichnet ist, daß zunächst auf beliebige, insbesondere auch herkömmliche Weise mit Wasser oder organischen Lösungsmitteln oder Gemischen davon ein alkoholisch-wässriger Primär- bzw. Rohextrakt (Flüssigextrakt) hergestellt wird, ― gegebenenfalls über den Umweg der Trockenextraktherstellung (zur Entfernung unerwünschter und für eine Ultrafiltration ungeeigneter Lösungsmittel) und der nachfolgenden Wiederaufnahme des Trockenextrakts in alkoholisch wässriger Lösung oder einem der anderen genannten Lösungsmittel, ― und daß dieser Flüssigextrakt dann einer gezielten Ultrafiltration über einen Filter mit einer durchschnittlichen Porengröße im Bereich von 2000 bis 10.000 Dalton unterworfen wird. Das flüssige Ultrafiltrat wird zwingend von organischen Lösungsmitteln befreit und wahlweise auch noch getrocknet. Für die Ultrafiltration werden vorzugsweise Filter aus Polyamid, Polypropylen oder regenerierter Cellulose eingesetzt. Besonders bevorzugt ist die Verwendung von Filtern mit etwa 3000 Dalton Porengröße.
Ohne abschließende Trocknung kann das von organischen Lösungsmitteln befreite Ultrafiltrat beispielsweise direkt zur Weiterverarbeitung in Arzneimitteln, Kosmetika und/oder diätischen Lebensmitteln eingesetzt werden.

Eine Variante des erfindungsgemäßen Verfahrens ist durch folgende Maßnahmen gekennzeichnet: Gewinnung eines Rohextrakts durch Extraktionsbehandlung der Blätter mit wässrig-alkoholischer Lösung, Entfernen des Extraktionslösungsmittels aus dem Rohextrakt, Verdünnung des auf diese Weise konzentrierten Rohextrakts durch Zugabe von Wasser, nachfolgende Kältebehandlung der wässrigen Lösung zur Ausfällung lipophiler Bestandteile, gegebenenfalls Versetzen des Überstands mit Alkohol oder einem anderen für eine Ultrafiltration geeigneten organischen Lösungsmittel zur Verbesserung des Auflösungsverhaltens der Extraktstoffe, anschließende Ultrafiltration dieser Lösung und abschließende Entfernung des/der organischen Lösungsmittel(s) und gegebenenfalls Trocknung des Ultrafiltrats.

Eine weitere Variante des erfindungsgemäßen Verfahrens ist durch die folgenden Schritte gekennzeichnet: Gewinnung eines Rohextrakts durch Extraktionsbehandlung der Blätter mit wässrig-alkoholischer Lösung, Entfernung des Extraktionslösungsmittels aus dem Rohextrakt, Verdünnung des auf diese Weise konzentrierten Rohextrakts durch Zugabe von Wasser, Kältebehandlung zur Ausfällung lipophiler Bestandteile, Entfernung von unerwünschten Inhaltsstoffen aus dem Überstand durch Fällungsreaktionen, Ad- und Desorptionsverfahren, Extraktion mit n-Butanol, o.ä. Reinigungsverfahren (vgl. DE 39 40 091, DE 30 40 092, US 5,637,302, J 279 300), gegebenenfalls Trocknung des derart aufgereinigten Extrakts zur Entfernung von für eine Ultrafiltration ungeeigneten Lösungsmitteln, Wiederaufnahme des getrockneten Extrakts in vorzugsweise alkoholisch-wässriger Lösung und schließlich Ultrafiltration dieses vorzugsweise alskoholisch-wässrigen Flüssigextrakts und abschließende Entfernung des/der organischen Lösungsmittel(s) und gegebenenfalls Trocknung des Ultrafiltrats.

Der mit dem erfindungsgemäßen Verfahren erzeugte Trockenextrakt aus Blättern von *Ginkgo biloba* besteht ausschließlich aus Inhaltsstoffen der Pflanzenteile, d.h. er enthält bezogen auf die Stoffzusammensetzung des Extrakts keine zusätzlichen Stoffe und ist insbesondere frei von Solubilisierungshilfsmittelzusätzen und/oder galenischen Hilfsstoffen. Er weist einen im Vergleich zur Droge signifikant höheren prozentualen Gehalt an Terpenlaktonen und Flavonglykosiden auf, und er ist in Wasser leicht löslich, d.h. nach dem "Europäischen Arzneibuch" 1997 ist er im Volumenverhältnis von mindenstens 1 Teil Extrakt zu 10 Teilen Wasser praktisch restlos lösbar, denn es entsteht dabei eine klare Lösung, die auch nach mehreren Stunden noch ungetrübt ist.

Der erfindungsgemäße Trockenextrakt enthält praktisch alle pharmazeutisch, kosmetisch und diätisch gewünschten Pflanzeninhaltsstoffe, vor allem Terpenlaktone und Flavonglykoside, und kann insbesondere auch ohne weiteres Prodelphinidine und andere Proanthocyanidine enthalten.

Ein solcher Trockenextrakt ist nicht nur in pharmazeutischen, sondern ebensogut auch in kosmetischen und diätischen Produkten sehr gut einsetzbar.

Eine besonders vorteilhafte Variante eines erfindungsgemäßen Trockenextrakts ist dadurch gekennzeichnet, daß sie einen Gehalt an:
Flavonglykosiden von mindestens 20 % (m/m),
Terpenlaktone von mindestens 5% (m/m), und
Ginkgolsäuren von höchstens 5 ppm
aufweist.

Eine andere, ebenfalls sehr vorteilhafte Variante des erfindungsgemäßen Trockenextrakts zeichnet sich dadurch aus, daß sie einen Gehalt an:
Flavonglykosiden von mindestens 22-27 % (m/m),
Terpenlaktonen von mindestens 5-7 % (m/m),
Ginkgoliden A, B, C von mindestens 2,8-3,4 % (m/m),
Bilobaliden von mindestens 2,6-3,2 % (m/m) und
Ginkgolsäuren von höchstens 5 ppm
aufweist. Diese Variante entspricht den Angaben der Monographie "Ginkgo biloba Trockenextrakt" der Komission E des ehemaligen Bundesgesundheitsamts der Bundesrepublik Deutschland.

Die vorstehend beschriebenen erfindungsgemäßen Trockenextrakte können demnach auf einfachste Weise dadurch erhalten werden, daß auf beliebige Weise erhaltene Rohextrakte oder teilweise oder weitgehend aufgereinigte Extrakte einem Ultrafiltrationsverfahren unterworfen und abschließend getrocknet werden. Mit anderen Worten:

Der erfindungsgemäße getrocknete Extrakt ist erhältlich durch Ultrafiltration eines auf herkömmliche Weise hergestellten Primär- bzw. Rohextrakts und abschließende Entfernung des/der organischen Lösungsmittel(s) und gegebenenfalls Trocknung des Ultrafiltrats.

Der Erfindung liegt die völlig überraschenden Erkenntnis zugrunde, daß allein durch die gezielte Ultrafiltration offenbar solche Extraktkomponenten, die die Wasserlöslichkeit von Trockenextrakten behindern, entfernt oder zumindest außer Kraft gesetzt werden, während die Zusammensetzung der gewünschten Inhaltsstoffgruppen des Extrakts im wesentlichen unverändert bleibt. Im Fall von *Ginkgo biloba* Trockenextrakten führt die Ultrafiltration dazu, daß selbst die als schwer löslich geltenden Ginkgolide komplett mit dem Wasser in Lösung gehen.
Die erfindungsgemäße Kombination von Eigenschaften des Extrakts, nämlich wasserloslich, nativ, ausschließlich aus Inhaltsstoffen der Pflanzenteile bestehend und insbesondere frei von Solubilisierungsmitteln und/oder galenischen Hilfsstoffen kann offenbar allein durch die Ultrafiltrationsbehandlung hervorgebracht werden
Diese Tatsache, daß durch eine rein technische Maßnahme ein Trockenextrakt erhalten werden kann, der komplett in Wasser löslich ist, ist umso erstaunlicher, als bisherige Löslichkeitsverbesserungen bei Trockenextrakten nur durch Zugabe von galenischen Hilfsstoffen oder Löslichkeitsvermittler erzielt werden konnten.
Das von organischen Lösungsmitteln befreite Ultrafiltrat kann erfindungsgemäß auch direkt, d.h. ohne abschließende Trocknung, zur Weiterverarbeitung z.B in Arzneimitteln, Kosmetika und/oder diätischen Lebensmitteln eingesetzt werden.

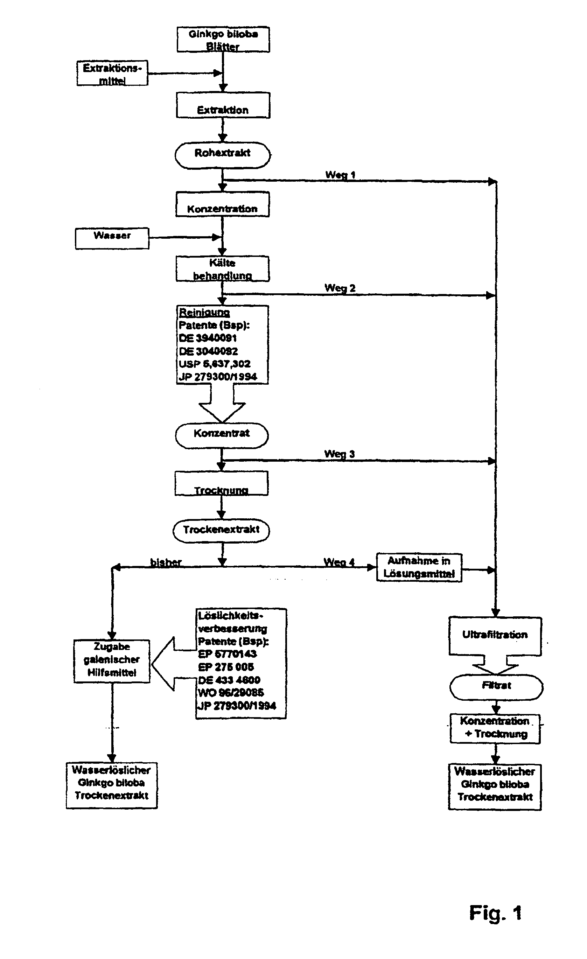

Die Erfindung wird im folgenden anhand einer graphischen Darstellung in Fig 1 und anhand von Ausführungsbeispielen näher erlautert.

**Fig. 1** zeigt am Beispiel von Ginkgo biloba die möglichen Herstellungswege zur Erzeugung von Ginkgo biloba Trockenextrakten der verschiedensten Reinheitsstufen Ausgangsmaterial ist in jedem Fall die Droge in Form von frischen oder getrockneten Ginkgo biloba Blättern
Aus diesen Blätter wird mit Hilfe eines alkoholisch-wässrigen oder ketonisch-wässrigen Lösungsmittels ein erster Extrakt, der Primär- oder Rohextrakt hergestellt

Dieser Rohextrakt kann bereits einer Ultrafiltration unterworfen und durch anschließende Trocknung in einen wasserlöslichen Trocken-Rohextrakt überführt werden (Weg 1)
Vielfach ist es jedoch notwendig oder wünschenswert, den Rohextrakt von unerwunschten Inhaltsstoffen zu reinigen, bevor er seiner Zweckbestimmung zugeführt wird Hierzu wird der Extrakt in der Regel zunächst von dem /den Extraktionslosungsmittel(n) (weitgehend) befreit, der so konzentrierte Rohextrakt durch Zugabe von Wasser wieder verdünnt, und diese wässrige Mischung durch Kältebehandlung einer Fällungsreaktion unterworfen, bei der vor allem lipophile Inhaltsstoffe ausfallen und abgetrennt werden. Dieser teilweise aufgereinigte, flüssige Extrakt kann dann ultrafiltriert und anschließend getrocknet werden, wobei ein wasserlöslicher, teilweise aufgereinigter Trockenextrakt erhalten wird (Weg 2)
Zur Herstellung relativ reiner Extrakte, bei denen die unerwunschten Inhaltsstoffe weitgehend entfernt und die gewünschten Inhaltsstoffe angereichert sind, wird jedoch dieser vorgereinigte bzw teilweise aufgereinigte flussige Extrakt noch weiteren Reinigungsverfahren unterworfen, beispielsweise Fällungsreaktionen wie in der DE 39 40 091 beschrieben oder Extraktionsverfahren mit n-Butanol wie in der DE 30 40 092 und der US 5.637.302, beschrieben, oder Ad- und Desorptionsverfahren wie in der J 279 300 beschrieben. Die mit diesen Reinigungsverfahren gewonnenen, weitgehend aufgereinigten Extrakte können dann entweder direkt, sofern sie als Flüssigextrakte vorliegen und das betreffende Lösungsmittel für ein Ultrafiltrationsverfahren geeignet ist (Weg 3), oder indirekt, nämlich über den Umweg der Konzentrierung und/oder Trocknung zur Entfernung ungeeigneter Lösungsmittel und der anschließenden Wiederverflüssigung durch Aufnahme in vorzugsweise wässrigalkoholischer Lösung (Weg 4), einer Ultrafiltration unterworfen und anschließend getrocknet werden. Das Endprodukt von Weg 3 und Weg 4 ist ein erfindungsgemäßer, wasserlöslicher, weitgehend aufgereinigter Trockenextrakt Der in der Graphik parallel zu Weg 4 dargestellte Weg "bisher" veranschaulicht die herausragende Einfachheit des erfindungsgemäßen Verfahrens im Vergleich zu den im Stand der Technik bekannten Verfahren zur Gewinnung von weitgehend aufgereinigten Trockenextrakten, die in Wasser loslich sind

### Ausführungsbeispiele

Bei samtlichen nachfolgend beschriebenen Beispielen gilt, daß die eingesetzten Ausgangsextrakte auf verschiedenen Herstellungswegen, insbesondere auch mit verschiedenen Extraktionsmitteln wie z B. mit Aceton, Ethanol oder Butanol hergestellt werden konnen

### Beispiel 1:

4,65 g Ginkgo-biloba EGb 761 Trockenextrakt werden mit 50 (m/m)%-igem Ethanol auf eine 10%-ige Lösung eingestellt und mittels Ultrafiltrationsanlage unter Verwendung einer Polyamid-Membran mit 5000 Dalton Porengröße filtriert. Das Retentat wird nochmals sechsmal mit 30 ml 60 (m/m)%-igem Ethanol nachgewaschen Die den Filter passierende Lösung (= Filtrat) wird konzentriert (z. B unter vakuum am Rotationsverdampfer) und im Vakuumtrockenschrank über Nacht bei 45°C und < 50 mbar getrocknet
Als Endergebnis erhält man 3,47g Filtrat (= 74,62 % Ausbeute) und 1,18g Retentat (= 25.38 % Ausbeute).

Die Extraktanalyse ergibt folgende Gehalte

| | gefundene Werte (Bsp 1) |
|---|---|
| Summe Terpenlaktone | 6,3 % |
| Flavonglykoside | 24,05 % |
| Ginkgolsäuren | <5 ppm |

Der Trockenextrakt aus dem Filtrat kann als 0,1%-ige Lösung in Wasser vollständig und klar gelöst werden. Die Lösung trübt innerhalb einer 2-stündigen Standzeit nicht nach Eine Filtration der Lösung über eine Filterschicht (Papierfilter; Porengröße 1 µm) resultiert in nur einem sehr geringen Filterrückstand von 0,12% der Extrakteinwaage (= Trockenextrakt in der Lösung).

### Beispiel 2:

1,3 kg *Ginkgo biloba* Blätter mit 1 % Flavonglykosiden und 0,26 % Terpenlaktonen werden mit insgesamt 10,5 kg 80(m/m)%igem Ethanol bei 60° C zweifach mit Hilfe einer Wirbelstromextraktion extrahiert Die Trennung der Rohextraktlösung mit den gelösten Bestandteilen von den ausextrahierten Drogenteilen erfolgt mit Hilfe einer Vakuumnutsche und einem Seitz Plattenfilter Nr. 1500.
Es resultieren 9,5 kg filtrierte Rohextraktlösung mit einem Feststoffgehalt von 3,85 % Die Lösung wird unter Vakuum schonend bei maximal 65° C Produkttemperatur im Verhältnis 12 zu 1 zu einem Konzentrat mit 44 % Trockenruckstand eingeengt
Unter Zugabe von demineralisiertem Wasser wird das Konzentrat auf 17 % Feststoffgehalt eingestellt und nachfolgend über Nacht bei 8° C gekühlt Die ausgefallten wasserunlöslichen Bestandteile werden über einen 1500 Seitz-Plattenfilter unter Zugabe von 107,6 g Filterhilfsmittel ("Filter Cel", Fa. Lehmann & Voss & Co., Hamburg) abfiltriert.
Es resultiert eine klare Extraktlösung von 2,4 kg mit einem Feststorfgehalt von 10%.

Die Extraktlösung wird über eine Säule mit 0,96 Liter Adsorberharz Diaion HP 20 von Mitsubishi Chemical gepumpt Nach Aufgabe des Extraktes erfolgt die Spülung der Säule mit 1,6 Liter demineralisiert Wasser und mit 3 Liter 60 (m/m)%igem Ethanol
Das 60 (m/m)%-ige Ethanol Desorbat der Säule mit einem Feststoffgehalt von 1,6 % (entspricht 48 g Trockenextrakt) wird direkt über eine Ultrafiltrationsanlage mit einer Polypropylen-Membran mit 5000 Dalton Porengröße (Firma Dow Danmark) filtriert Die Membran wird fünfmal mit 500 ml 60 (m/m)% Ethanol nachgewaschen. Das resultierende Filtrat wird schonend unter Vakuum konzentriert und über Nacht im Trockenschrank bei 45° C und < 50 mbar getrocknet. Es werden 36,44 g Trockenextrakt im Filtrat erhalten, was einer Ausbeute von 2,8%, bezogen auf die eingesetzte Menge an Droge entspricht.

| Extraktanalyse | |
|---|---|
| | gefundene Werte (Bsp. 2) |
| Summe Terpenlaktone | 6,95 % |
| Summe Ginkolide A, B, C | 3,5 1 % |
| Bilobalid | 3,44 % |
| Flavonglykoside | 26,73 % |
| Ginkgolsäuren | <5 ppm |

Für den Löslichkeitstest in Wasser wird 1 Teil Extrakt in 10 Teilen Wasser unter Rühren gelöst. Der Extrakt ist spontan klar gelöst und kann entsprechend dem "Europäischen Arzneibuch" 1997 als "leicht löslich" definiert werden Eine Filtration der Lösung über eine Filterschicht (Papierfilter; Porengröße 1 µm) resultiert in nur einem sehr geringen Filterrückstand von 0,18% der Extrakteinwaage (= Trockenextrakt in der Lösung).Vor der Ultrafiltration ist der Extrakt definitionsgemäß nach dem "Europäischen Arzneibuch" 1997 schwer loslich

### Beispiel 3:

98 g des unter 2.2 eingesetzten Extrakts werden in 1000 ml 60(m/m)%-igen Ethanol gelost und unter Verwendung einer Spiralpatrone S1 Y3 (Firma Amicon) aus regenerierter Cellulose mit 3000D Porengröße filtriert. Die Membran wird zweimal mit 500 ml des gleichen Lösungsmittels nachgewaschen
Das resultierende Filtrat wird anschließend schonend im Vakuum konzentriert und über Nacht im Trockenschrank bei 45°C und < 50 mbar getrocknet Man erhält 76,37g Trockenextrakt, das entspricht einer Ausbeute von 77,9%.

| Extraktanalyse. | |
|---|---|
| | gefundene Werte (Bsp 3) |
| Summe Terpenlaktone | 6,77 % |
| Summe Ginkolide A, B, C | 3,49 % |
| Bilobalid | 3,28 % |
| Flavonglykoside | 26,02 % |
| Ginkgolsäuren | <5 ppm |

Die Löslichkeit in Wasser ist gemäß dem "Europäischen Arzneibuch" 1997 als "leicht löslich" anzugeben (siehe Beispiel 2). Eine Filtration der Lösung über eine Filterschicht (Papierfilter; Porengröße 1 µm) erbringt nur einen sehr geringen Filterrückstand von 0,2% der Extrakteinwaage (= Trockenextrakt in der Losung).

### Beispiel 4:

100g eines nach der Lehre des deutschen Patents DE 39 40 092 hergestellten Ginkgo biloba Trockenextrakts werden in 1000 ml 60(m/m)%-igem Ethanol gelöst und unter Verwendung einer Polypropylenmembran mit einer Porengröße von 10000 Dalton ultrafiltriert. Die Membran wird zweimal mit 1000 ml des gleichen (vorgenannten) Lösungsmittels nachgewaschen. Das resultierende Filtrat wird schonend im Vakuum konzentriert und über Nacht bei einer Temperatur von 45°C und einem Druck von < 50 mbar getrocknet. Man erhält 82,64g Extrakt, dessen Gehalt an Terpenlaktonen, Ginkgoliden A,B und C, Bilobaliden, Flavonglykosiden und Ginkgolsauren im jeweils definierten Bereich gemäß der Monographie "Ginkgo biloba Trockenextrakt" der Komission E des ehemaligen Bundesgesundheitsamts der Bundesrepublik Deutschland liegt

6g dieses Trockenextrakts werden in 50g demineralisiertem Wasser gelöst. Die Löslichkeit in Wasser ist gemäß dem "Europäischen Arzneibuch" 1997 als "leicht löslich" anzugeben. Eine Filtration der Lösung über eine Filterschicht (Papierfilter; Porengröße 1 µm) erbringt nur einen sehr geringen Filterrückstand von 0,63% der Extrakteinwaage (= Trockenextrakt in der Lösung).

### Beispiel 5: Kontrollversuche

### a)

5 g *Ginkgo biloba* Trockenextrakt EGb 761 werden in 50 g dem. Wasser gelöst Die Löslichkeit in Wasser ist gemäß dem "Europäischen Arzneibuch" 1997 als schwer löslich anzugeben Die Filtration der Lösung über eine Filterschicht (Papierfilter; Porengröße 1 µm) erbringt Filterrückstände von durchschnittlich 18,2% der Extrakteinwaage (= Trockenextrakt in der Lösung).

### b)

5 g *Ginkgo biloba* Trockenextrakt, herstgestellt nach der Ethanolmethode (gemäß JP 279 300) und ohne Ultrafiltration, werden in 50 g demineralisiertes Wasser gelost Die Loslichkeit des Extraktes in Wasser ist gemäß dem "Europäischen Arzneibuch" 1997 als schwer löslich anzugeben. Die Filtration der Lösung über eine Filterschicht (Papierfilter; Porengröße 1 µm) erbringt Filterrückstände von durchschnittlich 15,2% der Extrakteinwaage (= Trockenextrakt in der Lösung)

## Patentansprüche

1. Verfahren zur Herstellung eines wasserlöslichen nativen Trockenextrakts aus Blättern von Ginkgo biloba, **gekennzeichnet durch** die Anzahl und Reihenfolge der folgenden Verfahrensschritte
(a) Herstellung eines Primär- bzw. Rohextrakts, nämlich eines alkoholisch- wässrigen Flüssigextrakts oder eines Trockenextrakts
gemäß beliebiger Verfahren;
(b) gegebenenfalls Aufnahme des Trockenextrakts in Wasser oder organischem Lösungsmittel oder Mischungen davon, vorzugsweise in alkoholisch-wässriger Lösung;
(c) Ultrafiltration der - vorzugsweise alkoholisch- wässerigen - Extrakt-Lösung über einen Filter mit durchschnittlicher Porengröße im Bereich von 2000 bis 10.000 Dalton;
(d) Entfernung des/der organischen Lösungsmittel(s) und gegebenenfalls Trocknung des Ultrafiltrats.

2. Verfahren nach Anspruch 1, **dadurch gekennzeichnet,**
**daß** die Herstellung des Trockenextrakts in Schritt (a) nach einem Verfahren mit der nachfolgend genannten Anzahl und Reihenfolge von Verfahrensschritte durchgeführt wird:
- Gewinnung eines Rohextrakts durch Extraktionsbehandlung der gewünschten Pflanzenteile mit wässrig-alkoholischer Lösung,
- Entfernung des Extraktionslösungsmittels,
- Entfernung unerwünschter, insbesondere lipophiler Inhaltsstoffe mittels Fällungsreaktion durch Wasserzugabe und Kältebehandlung,
- Durchführung weiterer Reinigungsverfahren, insbesondere Fällungsreaktionen,
Ad- und Desorptionsverfahren, Extraktionsverfahren o.ä., zur Entfernung weiterer unerwünschter Inhaltsstoffe und Anreicherung erwünschter Inhaltsstoffe ,
Entfernung des/der Lösungsmittel(s) und Trocknung.

3. Wasserlöslicher, nativer Trockenextrakt aus Blättern von Ginkgo biloba, **dadurch gekennzeichnet, daß** er einen im Vergleich zur Droge höheren prozentualen Gehalt an Terpenlaktonen und Flavonglykosiden aufweist und durch das Verfahren gemäß Anspruch 1 oder Anspruch 2 erhältlich ist.

4. Trockenextrakt nach Anspruch 3, **dadurch gekennzeichnet, daß** der Extrakt ein getrockneter Primärextrakt = Rohextrakt ist.

5. Trockenextrakt nach Anspruch 3, **dadurch gekennzeichnet, daß** der Extrakt ein gegenüber dem Rohextrakt teilweise aufgereinigter, nämlich von Extraktionslösungsmitteln und von in der Kälte in wässriger Lösung ausfallenden Bestandteilen befreiter Trockenextrakt ist.

6. Trockenextrakt nach Anspruch 3, **dadurch gekennzeichnet, daß** der Extrakt ein gegenüber dem Rohextrakt weitgehend aufgereinigter, nämlich von Extraktionslösungsmitteln, von in der Kälte in wässriger Lösung ausfallenden Bestandteilen und von durch Fällungsreaktionen, Ad- und Desorptionsverfahren, Extraktion mit n-Butanol o.ä. Reinigungsverfahren abtrennbaren unerwünschten Inhaltsstoffen befreiter Trockenextrakt ist.

7. Trockenextrakt nach Anspruch 3, **gekennzeichnet durch** einen Gehalt an
Flavonglykosiden von mindestens 20 % (m/m),
Terpenlaktone von mindestens 5 % (m/m), und
Ginkgolsäuren von höchstens 5 ppm.

8. Trockenextrakt nach Anspruch 3, **gekennzeichnet durch** einen Gehalt an
Flavonglykosiden von mindestens 22-27 % (m/m),
Terpenlaktonen von mindestens 5-7 % (m/m),
Ginkgoliden A, B, C von mindestens 2.8-3.4 % (m/m),
Bilobaliden von mindestens 2.6-3.2 % (m/m), und
Ginkgolsäuren von höchstens 5 ppm

9. Verwendung des Extrakts nach einem der Ansprüche 3 bis 8 zur Herstellung von Arzneimitteln, Kosmetika und/oder diätischen Lebensmitteln.

## Claims

1. Procedure for manufacturing a water-soluble native dry extract from leaves of *Ginkgo biloba,* **characterized by** the number and sequence of the following procedural steps:
(a) manufacture of a primary extract or raw extract, respectively, that is a hydroalcoholic liquid extract or a dry extract according to any procedure desired;
(b) if necessary, absorption of the dry extract in water or organic solvent or mixtures thereof, preferably in hydroalcoholic solution;
(c) ultrafiltration of the preferably hydroalcoholic extract solution through a filter with an average pore size ranging from 2000 to 10000 Daltons;
(d) removal of the organic solvent(s) and, if necessary, drying of the ultrafiltrate.

2. Procedure according to claim 1, **characterized by** the fact that the dry extract in step (a) is manufactured according to a procedure with the following specified number and sequence of procedural steps:
- generation of a raw extract via the extraction treatment of desired plant parts with a hydroalcoholic solution;
- removal of the extraction solvent;
- removal of undesired, in particular lipophilic constituents in a precipitation reaction through the addition of water and cold treatment;
- execution of additional purification procedures, in particular precipitation reactions, adsorption and desorption procedures, extraction procedures and the like to remove additional undesired constituents and enrich desired constituents, removal of the solvent(s) and drying.

3. Water-soluble, native dry extract from leaves of *Ginkgo biloba,* **characterized by** a higher percentage content of terpenlactones and flavonglycosides in comparison to the drug and by the fact that it is obtained by a procedure according to claim 1 or claim 2.

4. Dry extract according to claim 3, **characterized by** the fact that the extract is a dried primary extract = raw extract.

5. Dry extract according to claim 3, **characterized by** the fact that the extract is a partially purified dry extract in comparison with the raw extract, that is a dry extract freed of extraction solvents, and of constituents precipitated in aqueous solution in the cold.

6. Dry extract according to claim 3, **characterized by** the fact that the extract is a dry extract largely purified in comparison with the raw extract, that is a dry extract freed of extraction solvents, of constituents precipitating in aqueous solution in the cold, and of undesired contents that can be separated out via precipitation reactions, adsorption and desorption processes, extraction with n-butanol and similar purification procedures.

7. Dry extract according to claim 3, **characterized by** a content of
at least 20 % (m/m) flavonglycosides,
at least 5 % (m/m) terpenlactones and
at most 5 ppm ginkgolic acids.

8. Dry extract according to claim 3, **characterized by** a content of:
at least 22 - 27 % (m/m) flavonglycosides,
at least 5 - 7 % (m/m) terpenlactones,
at least 2.8 - 3.4 % (m/m) ginkgolides A, B, C,
at least 2.6 - 3.2 % (m/m) bilobalide, and
at most 5 ppm of ginkgolic acids.

9. Use of the extract according to one of claims 3 to 8 to manufacture pharmaceuticals, cosmetics and/or dietary foodstuffs.

## Revendications

1. Procédé de fabrication d'un extrait sec natif hydrosoluble de feuilles de ginkgo biloba, **caractérisé par** le nombre et la succession des étapes de procédé suivantes :
a) fabrication d'un extrait primaire ou brut, à savoir un d'extrait liquide dans un mélange alcool-eau ou d'un extrait sec selon un procédé quelconque ;
b) éventuellement reprise de l'extrait sec dans l'eau ou un solvant organique ou un mélange de ceux-ci, de préférence dans une solution eau-alcool ;
c) ultrafiltration de la solution d'extrait - de préférence eau-alcool - sur un filtre dont la taille des pores est comprise dans l'intervalle 2 000 à 10 000 Dalton ;
d) élimination du / des solvant(s) organique(s) et éventuellement séchage du filtrat obtenu par ultrafiltration.

2. Procédé selon la revendication 1, **caractérisé en ce que** la fabrication de l'extrait sec à l'étape a) est réalisée selon un procédé ayant le nombre et la succession suivante d'étapes de procédé :
➢ obtention d'un extrait brut par un traitement par extraction de la partie de la plante souhaitée avec une solution eau-alcool ;
➢ élimination du solvant d'extraction ;
➢ élimination des composants non souhaités, notamment lipophiles au moyen d'une réaction de précipitation en ajoutant de l'eau et en refroidissant ;
➢ réalisation d'autres procédés de purification, notamment de réactions de précipitation, de procédé d'adsorption et désorption, procédés d'extraction ou autres procédés similaires, pour éliminer d'autres substances non souhaitées et concentrer les substances souhaitées ;
➢ élimination du / des solvant(s) et séchage.

3. Extrait sec natif hydrosoluble à base de feuilles du ginkgo biloba, **caractérisé en ce qu'**il contient une teneur en pour-cent en terpènes de lactone et en glucosides de flavone plus élevée que celle de la drogue et qu'il peut être obtenu par le procédé selon la revendication 1 ou 2.

4. Extrait sec selon la revendication 3, **caractérisé en ce que** l'extrait est un extrait primaire sec = extrait brut sec.

5. Extrait sec selon la revendication 3, **caractérisé en ce que** par rapport à l'extrait brut, l'extrait est un extrait sec partiellement purifié, à savoir débarrassé des solvants d'extraction et des substances précipitant à froid dans les solvants aqueux.

6. Extrait sec selon la revendication 3, **caractérisé en ce que** par rapport à l'extrait brut, l'extrait est un extrait sec pratiquement entièrement purifié, à savoir débarrassé des solvants d'extraction, des substances précipitant à froid dans les solvants aqueux et des composants non souhaités pouvant être éliminés par des réactions de précipitation, des réactions d'adsorption et de désorption, par extraction avec du n-butanol ou des procédés de purifications semblables.

7. Extrait sec selon la revendication 3, **caractérisé en ce que** par une teneur en
➢ glucosides de flavone d'au moins 20 % (m/m),
➢ terpènes de lactone d'au moins 5 % (m/m) et
➢ acides ginkgoliques d'au plus 5 ppm.

8. Extrait sec selon la revendication 3, **caractérisé en ce que** par une teneur en
➢ glucosides de flavone d'au moins 22 à 27 % (m/m),
➢ terpènes de lactone d'au moins 5 à 7 % (m/m),
➢ ginkgolides A, B, C d'au moins 2,8 à 3,4 % (m/m),
➢ bilobalides d'au moins 2,6 à 3,2 % (m/m) et
➢ acides glinkgoliques d'au plus 5 ppm.

9. Utilisation de l'extrait selon l'une des revendications 3 à 8 pour la fabrication de médicaments, de produits cosmétiques et / ou de produits diététiques.
